(19) Europäisches Patentamt / European Patent Office / Office européen des brevets

(11) **EP 3 358 362 A1**

(12) **EUROPEAN PATENT APPLICATION**

(43) Date of publication:
**08.08.2018 Bulletin 2018/32**

(51) Int Cl.:
*G01R 33/48* *(2006.01)*

(21) Application number: **18154464.4**

(22) Date of filing: **31.01.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **01.02.2017 KR 20170014384**

(71) Applicant: **Samsung Electronics Co., Ltd.
Gyeonggi-do 16677 (KR)**

(72) Inventors:
• **Suh, Hyun-sang
  Suwon-si, Gyeonggi-do (KR)**
• **Choi, Joon-sung
  Dongan-gu, Anyang-si, Gyeonggi-do (KR)**

(74) Representative: **Gulde & Partner
Patent- und Rechtsanwaltskanzlei mbB
Wallstraße 58/59
10179 Berlin (DE)**

(54) **MRI WITH SEPARATION OF FAT AND WATER SIGNALS**

(57) A method and apparatus for acquiring a magnetic resonance (MR) signal are provided. The method includes: applying one radio frequency (RF) excitation pulse to a region of interest that has undergone a frequency shift by injecting a contrast agent into an object; acquiring a plurality of echo signals at a plurality of different echo times based on phase differences among water, fat, and the region of interest; and separating, based on the acquired plurality of echo signals, MR signals respectively originating from the water, the fat, and the region of interest.

EP 3 358 362 A1

**Description**

CROSS-REFERENCE TO RELATED APPLICATION

This application is based on and claims priority under 35 U.S.C. §119 to Korean Patent Application No. 10-2017-0014384, filed on February 1, 2017, in the Korean Intellectual Property Office, the disclosure of which is incorporated by reference herein in its entirety.

BACKGROUND

**1. Field**

The disclosure relates to methods and apparatuses for acquiring magnetic resonance (MR) signals, and more particularly, to methods and apparatuses for separating MR signals with respect to water, fat, and a part of interest of an object based on a Dixon method.

**2. Description of Related Art**

A magnetic resonance imaging (MRI) apparatus uses a magnetic field to capture an image of an object. The MRI apparatus is used in disease diagnosis because stereoscopic images of bones, lumbar discs, joints, nerve ligaments, the heart, etc. can be obtained at desired angles. In a contrast imaging technique whereby a contrast agent is injected during acquisition of an MR image, imaging sequences need to be performed twice, i.e., both before and after injection of the contrast agent, to obtain an MR image of a part of interest. This may increase the overall scan time. Another drawback is that spatial distribution varies between tissues in an object due to patient movement that occurs before and after injection of the contrast agent, which may make imaging diagnosis difficult. In particular, when abdominal contrast imaging is performed, an examinee is forced to stop breathing during the imaging. In this case, a change in position occurs over a long period of time due to different respiratory cycles and may make diagnosis difficult when comparing images generated before and after administration of the contrast agent, particularly when the images are acquired at different times and locations.

Thus, it is desirable to obtain an MR image of an object into which a contrast agent is injected in a single scan. A currently known method is a Dixon technique whereby separate MR images of water and fat are acquired by individually separating water and fat, based on a frequency difference between the water and fat, after application of a radio frequency (RF) pulse.

SUMMARY

Embodiments of the disclosure relate to methods and apparatuses for separating magnetic resonance (MR) signals with respect to water, fat, and a part of interest of an object into which a contrast agent is injected by using a Dixon method.

In accordance with an aspect of the disclosure, a method of acquiring a magnetic resonance (MR) image by injecting a contrast agent into a region of interest of an object includes applying one radio frequency (RF) excitation pulse to the region of interest after the region of interest has undergone a frequency shift caused by the injected contrast agent, acquiring a plurality of echo signals at a plurality of echo times, the plurality of echo times being determined based on phase differences among water, fat, and the region of interest, and separating, based on the acquired plurality of echo signals, MR signals originating respectively from the water, the fat, and the region of interest.

The acquiring of the plurality of echo signals may further include acquiring at least three echo signals across at least three echo times.

The acquiring of the plurality of echo signals may further include determining the plurality of echo times at which phase differences caused by frequency differences among the water, the fat, and the region of interest reach predetermined phase difference values, and acquiring the plurality of echo signals at the calculated plurality of echo times.

The determining of the plurality of echo times may further include: determining a first echo time at which a phase difference between the water and the fat is equal to a first phase angle, determining a second echo time at which a phase difference between the water and the region of interest is equal to a second phase angle, determining a third echo time at which the phase difference between the water and the region of interest is equal to a phase difference between the fat and the region of interest, and determining a fourth echo time at which the water, the fat, and the region of interest are all in phase.

The determining of the plurality of echo times may further include determining the first echo time as an echo time at which the first phase angle is $\pi$.

The determining of the plurality of echo times may further include determining the second echo time as an echo time at which the second phase angle is $\pi$.

The determining of the plurality of echo times may further include determining the third echo time as an echo time at which the phase difference between the water and the region of interest is equal to $\pi/2$ and the phase difference between the fat and the region of interest is equal to $\pi/2$.

The method may further include repeating the applying of the one RF excitation pulse and the acquiring of the plurality of echo signals a plurality of times.

The separating of the MR signals may further include generating complex image data of the water, complex image data of the fat, and complex image data of the region of interest by reconstructing the acquired plurality of echo signals, generating a signal equation for individually separating the complex image data of the water, the complex image data of the fat, and the complex image data of the region of interest, and eliminating the complex image data of the water and the complex image data of the fat based on the signal equation, and acquiring the complex image data of the region of interest.

The method may further include reconstructing an MR image of the region of interest from the complex image data of the region of interest.

In accordance with an aspect of the disclosure, an apparatus for acquiring a magnetic resonance (MR) image by injecting a contrast agent into a region of interest of an object includes a radio frequency (RF) coil configured to apply one RF excitation pulse to the region of interest after the region of interest has undergone a frequency shift caused by the injected contrast agent, a controller configured to determine a plurality of echo times based on phase differences among water, fat, and the region of interest, and an image processor configured to acquire a plurality of echo signals at the plurality of echo times and separate, based on the acquired plurality of echo signals, MR signals originating respectively from the water, the fat, and the region of interest.

The image processor may be further configured to acquire at least three echo signals across at least three echo times.

The controller may be further configured to calculate the plurality of echo times at which phase differences caused by frequency differences among the water, the fat, and the region of interest reach predetermined phase difference values, and the image processor may be further configured to acquire the plurality of echo signals at the calculated plurality of echo times.

The controller may be further configured to determine a first echo time at which a phase difference between the water and the fat is equal to a first phase angle, determine a second echo time at which a phase difference between the water and the region of interest is equal to a second phase angle, determine a third echo time at which the phase difference between the water and the region of interest is equal to a phase difference between the fat and the region of interest, and determine a fourth echo time at which the water, the fat, and the region of interest are all in phase.

The controller may be further configured to determine the first echo time as an echo time at which the first phase angle is $\pi$.

The controller may be further configured to determine the second echo time as an echo time at which the second phase angle is $\pi$.

The controller may be further configured to determine the third echo time as an echo time at which the phase difference between the water and the region of interest is equal to $\pi/2$ and the phase difference between the fat and the region of interest is equal to TT/2.

The RF coil may be further configured to repeatedly apply the one RF excitation pulse a plurality of times and the controller may be further configured to repeatedly acquire the plurality of echo signals a plurality of times.

The image processor may be further configured to generate complex image data of the water, complex image data of the fat, and complex image data of the region of interest by reconstructing the acquired plurality of echo signals, generate a signal equation for individually separating the complex image data of the water, the complex image data of the fat, and the complex image data of the region of interest, acquire the complex image data of the region of interest by eliminating the complex image data of the water and the complex image data of the fat based on the signal equation, and reconstruct an MR image of the region of interest from the complex image data of the region of interest.

In accordance with an aspect of the disclosure, a non-transitory computer-readable recording medium having recorded thereon at least one program which, when executed by a processor, causes the processor to perform the methods above.

BRIEF DESCRIPTION OF THE DRAWINGS

The above and other aspects, features, and advantages of certain embodiments of the present disclosure will become apparent from the following description taken in conjunction with the accompanying drawings, in which:

FIG. 1 is a schematic diagram of a magnetic resonance imaging (MRI) system according to an embodiment;

FIG. 2 is a block diagram of a configuration of an MRI apparatus according to an embodiment;

FIG. 3 is a flowchart of a method, performed by an MRI apparatus, of acquiring an MR signal, according to an embodiment;

FIG. 4 illustrates a frequency spectrum for explaining a frequency shift in an object caused by injection of a contrast agent;

FIGS. 5A and 5B are timing diagrams for pulse sequences performed by an MRI apparatus to acquire echo signals, according to embodiments;

FIG. 6 is a schematic diagram for explaining phase differences among water, fat, and an object, in pieces of echo data respectively acquired by an MRI apparatus at a plurality of echo times, according to an embodiment;

FIG. 7 is a flowchart of a method, performed by an MRI apparatus, of acquiring a plurality of echo signals, according to an embodiment;

FIG. 8 is a schematic diagram for explaining phase differences among water, fat, and an object, in pieces of echo data respectively acquired by an MRI apparatus at a plurality of echo times, according to an embodiment; and

FIG. 9 is a flowchart of a method, performed by an MRI apparatus, of generating an MR image of an object, according to an embodiment.

## DETAILED DESCRIPTION

The present specification describes principles of the present disclosure and sets forth embodiments thereof to clarify the scope of the present disclosure and to allow those of ordinary skill in the art to implement the embodiments. The present embodiments may have different forms.

Like reference numerals refer to like elements throughout. The present specification does not describe all components in the embodiments, and common knowledge in the art or the same descriptions of the embodiments will be omitted below. The term "part" or "portion" may be implemented using hardware or software, and according to embodiments, one "part" or "portion" may be formed as a single unit or element or include a plurality of units or elements. Hereinafter, the principles and embodiments of the present disclosure will be described in detail with reference to the accompanying drawings. Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, or all of a, b, and c.

In the present specification, an "image" may include a medical image obtained by a magnetic resonance imaging (MRI) apparatus, a computed tomography (CT) apparatus, an ultrasound imaging apparatus, an X-ray apparatus, or another medical imaging apparatus.

Furthermore, in the present specification, an "object" may be a target to be imaged and include a human, an animal, or a part of a human or animal. For example, the object may include a body part, for example, an organ, or a phantom.

In the present specification, a 'part of interest' may refer to a specific part that a user, for example, a medical doctor or radiologist, desires to observe within an object. For example, the part of interest may be a particular tissue, organ or cell of the object, or a phantom.

In one or more embodiments, an MRI system acquires an MR signal and reconstructs the acquired MR signal into an image. The MR signal denotes a radio frequency (RF) signal emitted from the object.

In the MRI system, a main magnet creates a static magnetic field to align a magnetic dipole moment of a specific atomic nucleus of the object placed in the static magnetic field along a direction of the static magnetic field. A gradient coil may generate a gradient magnetic field by applying a gradient signal to a static magnetic field and induce resonance frequencies differently according to each region of the object.

An RF coil may emit an RF signal to match a resonance frequency of a region of the object whose image is to be acquired. Furthermore, when gradient magnetic fields are applied, the RF coil may receive MR signals having different resonance frequencies emitted from a plurality of regions of the object. Though this process, the MRI system may obtain an image from an MR signal by using an image reconstruction technique.

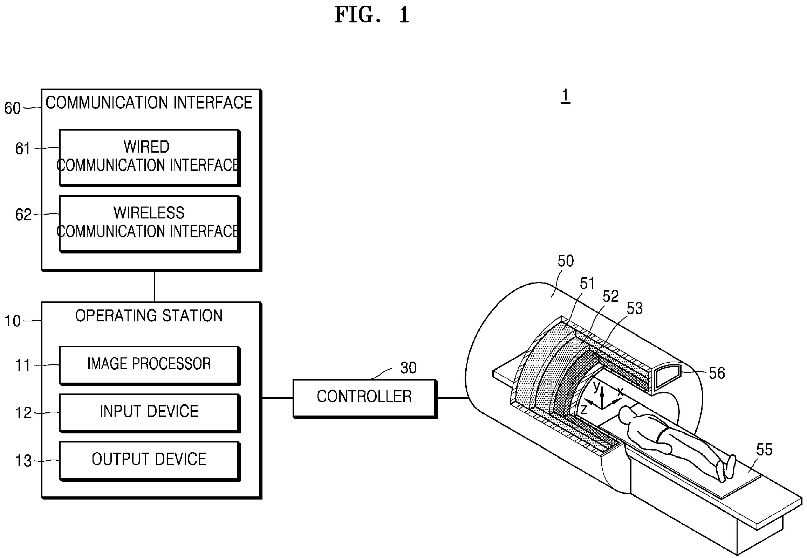

FIG. 1 is a schematic diagram of an MRI system 1. Referring to FIG. 1, the MRI system 1 may include an operating station 10, a controller 30, and a scanner 50. The controller 30 may be independently separated from the operating station 10 and the scanner 50. Furthermore, the controller 30 may be separated into a plurality of sub-components and incorporated into the operating station 10 and the scanner 50 in the MRI system 1. Operations of the components in the MRI system 1 will now be described in detail.

The scanner 50 may be formed to have a cylindrical shape, for example, a shape of a bore, having an empty inner space into which an object may be inserted. A static magnetic field and a gradient magnetic field are created in the inner space of the scanner 50, and an RF signal is emitted toward the inner space.

The scanner 50 may include a static magnetic field generator 51, a gradient magnetic field generator 52, an RF coil 53, a table 55, and a display 56. The static magnetic field generator 51 creates a static magnetic field for aligning magnetic dipole moments of atomic nuclei of the object in a direction of the static magnetic field. The static magnetic field generator 51 may be formed as a permanent magnet or superconducting magnet using a cooling coil.

The gradient magnetic field generator 52 is connected to the controller 30 and generates a gradient magnetic field by applying a gradient to a static magnetic field in response to a control signal received from the controller 30. The gradient magnetic field generator 52 includes X, Y, and Z coils for generating gradient magnetic fields in X-, Y-, and Z-axis directions crossing each other at right angles and generates a gradient signal according to a position of a region being imaged so as to differently induce resonance frequencies according to regions of the object.

The RF coil 53 connected to the controller 30 may emit an RF signal toward the object in response to a control signal received from the controller 30 and receive an MR signal emitted from the object. In detail, the RF coil 53 may transmit, toward atomic nuclei of the object having precessional motion, an RF signal having the same frequency as that of the precessional motion, stop transmitting the RF signal, and then receive an MR signal emitted from the object.

The RF coil 53 may be formed as a transmitting RF coil for generating an electromagnetic wave having an RF corresponding to the type of an atomic nucleus, a receiving RF coil for receiving an electromagnetic wave emitted from an atomic nucleus, or one transmitting/receiving RF coil serving both functions of the transmitting RF coil and receiving RF coil. Furthermore, in addition to the RF coil 53, a separate coil may be attached to the object. Examples of the separate coil may include a head coil, a spine coil, a torso coil, and a knee coil according to a region being imaged or to which the separate coil is attached.

The display 56 may be disposed outside and/or inside the scanner 50. The display 56 is also controlled by the controller 30 to provide a user or the object with information related to medical imaging.

Furthermore, the scanner 50 may include an object monitoring information acquisition unit configured to acquire and transmit monitoring information about a state of the object. For example, the object monitoring information acquisition unit may acquire monitoring information related to the object from a camera for capturing images of a movement or position of the object, a respiration measurer for measuring the respiration of the object, an ECG measurer for measuring the electrical activity of the heart, or a temperature measurer for measuring a temperature of the object and transmit the acquired monitoring information to the controller 30. The controller 30 may in turn control an operation of the scanner 50 based on the monitoring information. Operations of the controller 30 will now be described in more detail.

The controller 30 may control overall operations of the scanner 50.

The controller 30 may control a sequence of signals formed in the scanner 50. The controller 30 may control the gradient magnetic field generator 52 and the RF coil 53 according to a pulse sequence received from the operating station 10 or a designed pulse sequence.

A pulse sequence may include all pieces of information required to control the gradient magnetic field generator 52 and the RF coil 53. For example, the pulse sequence may include information about a strength, a duration, and application timing of a pulse signal applied to the gradient magnetic field generator 52.

The controller 30 may control a waveform generator for generating a gradient wave, i.e., an electrical pulse according to a pulse sequence and a gradient amplifier for amplifying the generated electrical pulse and transmitting the same to the gradient magnetic field generator 52. Thus, the controller 30 may control formation of a gradient magnetic field by the gradient magnetic field generator 52.

Furthermore, the controller 30 may control an operation of the RF coil 53. For example, the controller 30 may supply an RF pulse having a resonance frequency to the RF coil 53 that emits an RF signal toward the object, and receive an MR signal received by the RF coil 53. In this case, the controller 30 may adjust emission of an RF signal and reception of an MR signal according to an operating mode by controlling an operation of a switch, for example, a T/R switch, for adjusting transmitting and receiving directions of the RF signal and the MR signal based on a control signal.

The controller 30 may control a movement of the table 55 where the object is placed. Before MRI is performed, the controller 30 may move the table 55 according to which region of the object is to be imaged.

The controller 30 may also control the display 56. For example, the controller 30 control the on/off state of the display 56 or a screen to be output on the display 56 according to a control signal.

The controller 30 may be formed as an algorithm for controlling operations of the components in the MRI system 1, a memory for storing data in the form of a program, and a processor for performing the above-described operations by using the data stored in the memory. In this case, the memory and the processor may be implemented as separate chips. Alternatively, the memory and processor may be incorporated into a single chip.

The operating station 10 may control overall operations of the MRI system 1 and include an image processor 11, an input device 12, and an output device 13.

The image processor 11 may control the memory to store an MR signal received from the controller 30, and generate image data with respect to the object from the stored MR signal by applying an image reconstruction technique by using an image processor.

For example, if a k space, for example, also referred to as a Fourier space or a frequency space, of the memory is filled with digital data to complete k-space data, the image processor 11 may reconstruct image data from the k-space data by applying various image reconstruction techniques, for example, by performing inverse Fourier transform on the k-space data, by using the image processor.

Furthermore, the image processor 11 may perform various signal processing operations on MR signals in parallel. For example, image processor 11 may perform signal processing on a plurality of MR signals received via a multi-channel RF coil in parallel so as to convert the plurality MR signals into image data. In addition, the image processor 11 may store not only the image data in the memory, or the controller 30 may store the same in an external server via a communication interface 60 as will be described below.

The input device 12 may receive, from the user, a control command for controlling the overall operations of the MRI system 1. For example, the input device 12 may receive, from the user, object information, parameter information, a scan condition, and information about a pulse sequence. The input device 12 may be a keyboard, a mouse, a track ball, a voice recognizer, a gesture recognizer, a touch screen, or any other input device.

The output device 13 may output image data generated by the image processor 11. The output device 13 may also output a user interface (UI) configured so that the user may input a control command related to the MRI system 1. The output device 13 may be formed as a speaker, a printer, a display, or any other output device.

Furthermore, although FIG. 1 shows that the operating station 10 and the controller 30 are separate components, the operating station 10 and the controller 30 may be included in a single device as described above. Furthermore, processes respectively performed by the operating station 10 and the controller 30 may be performed by another component. For example, the image processor 11 may convert an MR signal received from the controller 30 into a digital signal, or the controller 30 may directly perform the conversion of the MR signal into the digital signal.

The MRI system 1 may further include a communication interface 60 and be connected to an external device such as a server, a medical apparatus, and a portable device, for example, a smartphone, a tablet PC, a wearable device, etc., via the communication interface 60.

The communication interface 60 may include at least one component that enables communication with an external device. For example, the communication interface 60 may include at least one of a local area communication interface , a wired communication interface 61, and a wireless communication interface 62.

The communication interface 60 may receive a control signal and data from an external device and transmit the received control signal to the controller 30 so that the controller 30 may control the MRI system 1 according to the received signal.

Alternatively, by transmitting a control signal to an external device via the communication interface 60, the controller 30 may control the external device according to the control signal.

For example, the external device may process data of the external device according to a control signal received from the controller 30 via the communication interface 60.

A program for controlling the MRI system 1 may be installed on the external device and may include instructions for performing some or all of the operations of the controller 30.

The program may be preinstalled on the external device, or a user of the external device may download the program from a server providing an application for installation. The server providing an application may include a recording medium having the program recorded thereon.

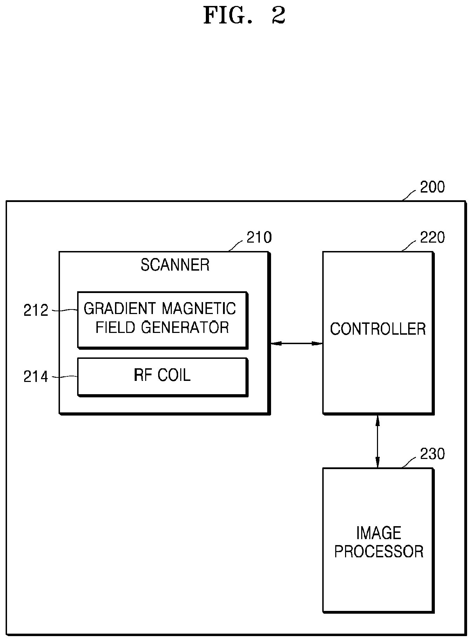

FIG. 2 is a block diagram of a configuration of an MRI apparatus 200 according to an embodiment.

Referring to FIG. 2, the MRI apparatus 200 according to the present embodiment may include a scanner 210 and a controller 220. The scanner 210 may include a gradient magnetic field generator 212 and an RF coil 214. Except for features of embodiments that will be described below, the scanner 210 and the controller 30 may respectively be the same components as the scanner 50 and the controller 30. Although FIG. 2 illustrates some components of the MRI apparatus 200, the MRI apparatus 200 may further include other components described with reference to FIG. 1.

The gradient magnetic field generator 212 may include gradient magnetic field coils and apply gradient magnetic fields in three-axis directions, i.e., X-, Y-, and Z-axis directions to an object based on a signal from a gradient magnetic field power supply. An imaging slice of the object may be set by adding together the applied gradient magnetic fields in the X-, Y-, and Z-axis directions.

The RF coil 214 may apply an RF excitation pulse to a part of interest in the object. The part of interest may be a part or region of the object that a user desires to observe, such as a specific tissue, organ or cell within the object, or a phantom.

The RF excitation pulse applied by the RF coil 214 may generate an echo signal that is a nuclear magnetic resonance (NMR) signal due to a phenomenon induced by nuclei of atoms constituting a tissue or cell in a part of interest, and the RF coil 214 may receive the echo signal. The RF coil 214 may include a transmitting RF coil for generating an electromagnetic wave having an RF corresponding to the type of an atomic nucleus and a receiving RF coil for receiving an electromagnetic wave emitted from an atomic nucleus. However, embodiments are not limited thereto. In other words, the RF coil 214 may include a transmitting RF coil and a receiving RF coil that are separately implemented.

According to an embodiment, the RF coil 214 may apply one RF excitation pulse to a part of interest that has undergone a frequency shift due to a contrast agent injected into the object and receive a plurality of different echo signals respectively at a plurality of echo times (TEs).

The controller 220 may control the gradient magnetic field generator 212 and the RF coil 214 to repeatedly generate a slice encoding gradient, a phase encoding gradient, a frequency encoding gradient, and an RF excitation pulse based on a preset pulse sequence.

The controller 220 may include a memory for pre-storing various pulse sequences as a program. According to an embodiment, the controller 220 may include hardware including a memory for storing at least one of a program, an algorithm, and data, as well as a pulse sequence, and a processor for processing a program, an algorithm, or data stored in the memory. For example, the controller may be composed of a processor including at least one of a central processing unit (CPU), a microprocessor, and a graphic processing unit.

According to an embodiment, the controller 220 may store a pulse sequence based on a Dixon method. The controller 220 may determine a plurality of different TEs based on phase differences among water, fat, and a part of interest into which a contrast agent is injected, and store pulse sequences for acquiring echo signals at the determined plurality of different TEs.

According to an embodiment, the controller 220 may calculate a plurality of TEs when phase differences respectively caused by frequency differences among water, fat, and a part of interest of an object reach their corresponding predetermined phase difference values. For example, the controller 220 may calculate a TE at which a phase difference between water and fat is equal to a predetermined first phase angle and determine the calculated TE as a first TE. Furthermore, the controller 220 may calculate a TE at which a phase difference between water and a part of interest is equal to a predetermined second phase angle and determine the calculated TE as a second TE. Furthermore, the controller 220 may calculate a TE at which a phase difference between the water and the part of interest is equal to a phase difference between the fat and the part of interest and determine the calculated TE as a third TE. In addition, the controller 220 may calculate a TE at which the water, the fat, and the part of interest are all in phase and determine the calculated TE as a fourth TE.

An image processor 230 may acquire a plurality of echo signals at a plurality of TEs determined by the controller 220. The image processor 230 may acquire at least three (3) echo signals across at least three TEs.

In an embodiment, the image processor 230 may individually separate pieces of image data regarding water, fat, and a part of interest of an object by combining the acquired plurality of echo signals. According to an embodiment, the image processor 230 may individually separate pieces of complex image data regarding the water, the fat, and the part of interest of the object based on the acquired plurality of echo signals. The image processor 230 may respectively reconstruct MR images of the water, the fat, and the part of interest from the separate pieces of complex image data.

The image processor 230 may be formed as a processor including at least one of a CPU, a microprocessor, and a graphic processing unit. However, embodiments are not limited thereto, and the image processor 230 may be implemented as an application specific integrated circuit (ASIC) or a field programmable gate array (FPGA).

Although FIG. 2 illustrates the controller 220 and the image processor 230 as separate from each other, embodiments are not limited thereto. In an embodiment, the controller 220 and the image processor 230 may be integrated into a signal processor.

In a conventional method of acquiring an MR image by using a contrast agent, imaging sequences need to be performed twice to acquire images before and after administration of a contrast agent, which may increase the overall scan time. Another drawback is that spatial distribution varies between tissues in an object due to movement of an examinee that occurs before and after injection of a contrast agent. In particular, when abdominal contrast imaging is performed, an examinee is forced to stop breathing during the imaging. In this case, a change in position occurs over a long period of time due to different respiratory cycles and may make diagnosis difficult by comparing images generated before and after administration of a contrast agent based on images acquired at different times and locations.

A Dixon method is known as a method of acquiring an MR image of a part of interest in an object into which a contrast agent is injected in a single scan. The MRI apparatus 200 of FIG. 2 may receive a plurality of echo signals at a plurality of TEs based on phase differences among water, fat, and a part of interest after administration of a contrast agent to an object and separate MR signals respectively originating from the water, the fat, and the part of interest individually by combining the received plurality of echo signals. The MRI apparatus 200 may eliminate MR signals from the water and fat and acquire only an MR signal from the part of interest.

The MRI apparatus 200 may be configured to generate an MR image before injection of a contrast agent based on the same MR image generated after injection of the contrast agent, thereby decreasing the overall scan time. Furthermore, the MRI apparatus 200 is configured to compare MR images generated based on the same MR image before and after injection of the contrast agent, thereby reducing side effects, for example motion artifacts, caused by movement of an examinee.

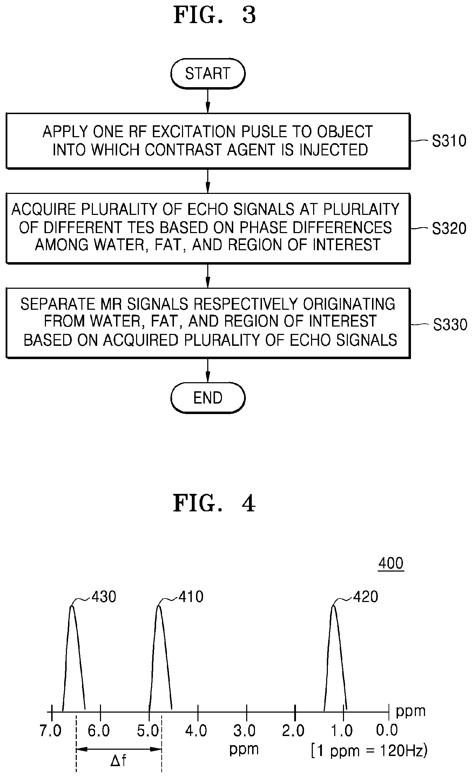

FIG. 3 is a flowchart of a method, performed by the MRI apparatus , for example MRI apparatus 200 of FIG. 2, of acquiring an MR signal according to an embodiment.

The MRI apparatus 200 applies one RF excitation pulse to a part of interest of an object into which a contrast agent is injected at operation S310. According to an embodiment, the controller 220 may control the RF coil 214 to generate an RF excitation pulse based on a preset pulse sequence.

The MRI apparatus 200 acquires a plurality of echo signals at a plurality of different TEs based on phase differences among water, fat, and the part of interest of the object at operation S320. When a contrast agent is injected into the object, a frequency shift occurs in the part of interest of the object. The controller 220 may determine a plurality of different TEs based on phase differences among water, fat, and the part of interest where a frequency shift occurs, respectively induced by frequency differences among the part of interest, the water, and the fat. The controller 220 may control the image processor 230 to acquire a plurality of echo signals based on the determined plurality of TEs.

According to an embodiment, the MRI apparatus 200 may acquire at least three echo signals across at least three TEs.

The MRI apparatus 200 may individually separate MR signals respectively originating from the water, the fat, and the part of interest based on the acquired plurality of echo signals at operation S330. According to an embodiment, the image processor 230 may convert the plurality of echo signals acquired in operation S320 into a plurality of pieces of complex image data. Furthermore, the image processor 230 may respectively separate the plurality of pieces of complex image data into complex image data of the water, complex image data of the fat, and complex image data of the part of interest by using signal equations.

FIG. 4 illustrates a frequency spectrum 400 for explaining a frequency shift in an object caused by injection of a contrast agent.

Referring to the frequency spectrum 400 shown in FIG. 4, there is a difference in resonance frequencies of peaks of water 410, fat 420, and a part of interest 430 at 3T. In the frequency spectrum 400, the resonance frequency of the part of interest 430 is obtained after a frequency shift is induced by a contrast agent injected therein.

For example, if a main magnetic field has a frequency of 0 Hz, resonance frequencies of the water 410, the fat 420, and the part of interest 430 have peak values at about 4.8 ppm (576 Hz), about 1.2 ppm (144 Hz), and about 6.6 ppm (792 Hz), respectively. However, the above-described resonance frequencies are merely an example, and resonance frequencies of the water 410, the fat 420, and the part of interest 430 are not limited thereto.

$T_1$ and $T_2$ values may vary according to the type and concentration of a contrast agent injected into a part of interest, and may be determined based on Equation (1) below:

$$\frac{1}{T_i'} = \frac{1}{T_i} + r_i \cdot C \quad i = 1,2 \qquad \ldots (1)$$

where $r_1$ and $r_2$ represent relaxivities of the contrast agent.

A resonance frequency shift $\Delta f$ in the part of interest into which the contrast agent is injected may be determined based on the following Equations (2) and (3) that will respectively be applied when shapes of the part of interest are spherical and cylindrical:

$$\Delta f_{sphere} = \frac{4}{3}\pi \cdot f_0 \cdot \chi_m \cdot C \qquad \ldots (2)$$

$$\Delta f_{cylinder} = 2\pi \cdot f_0 \cdot \chi_m \cdot C \cdot \left(cos^2\theta - \frac{1}{3}\right) \qquad \ldots (3)$$

where C is the concentration of the injected contrast agent and $\chi_m$ is a molar susceptibility of the contrast agent.

Examples of the contrast agent injected into the part of interest include Gadolinium-DOTA (Gd-DOTA) and Dysprosium-DOTA (Dy-DOTA). Tables 1 and 2 below respectively show the relaxivities with respect to the type of contrast agent at 3T and the extent of a frequency shift based on a molar susceptibility with respect to the type of contrast agent. However, values listed in Tables 1 and 2 are merely examples.

[Table 1]

| Type of contrast agent | $r_1$(s$^{-1}$ mM$^{-1}$) | $r_2$(s$^{-1}$mM$^{-1}$) | $\chi_m$ (cm$^3$mol$^{-1}$) |
|---|---|---|---|
| Gd-DOTA | 3.85 | 4.95 | 0.027 |
| Dy-DOTA | 0.121 | 0.132 | 0.048 |

[Table 2]

| Type of contrast agent | $T'_1$(ms) | $T'_2$(ms) | Frequency shift ($\Delta f_{sphere}$, @3T |
|---|---|---|---|
| 5mM Gd-DOTA | 50 | 32 | 72 |
| 5mM Dy-DOTA | 695 | 136 | 128 |
| 10mM Gd-DOTA | 25 | 18 | 144 |
| 10mM Dy-DOTA | 489 | 125 | 256 |

$T_1$ and $T_2$ values before injection of contrast agent into a part of interest of an object are 1200 and 150, respectively. As shown in Table 2, since $T_1$ and $T_2$ values when using the Gd-DOTA contrast agent vary to a greater extent than those when using the Dy-DOTA contrast agent, the Gd-DOTA contrast agent exhibits excellent relaxation enhancer functionality. Furthermore, since a frequency shift occurs to a greater extent when using the Dy-DOTA contrast agent than when using the Gd-DOTA contrast agent, it can be seen that the Dy-DOTA contrast agent exhibits excellent susceptibility effects.

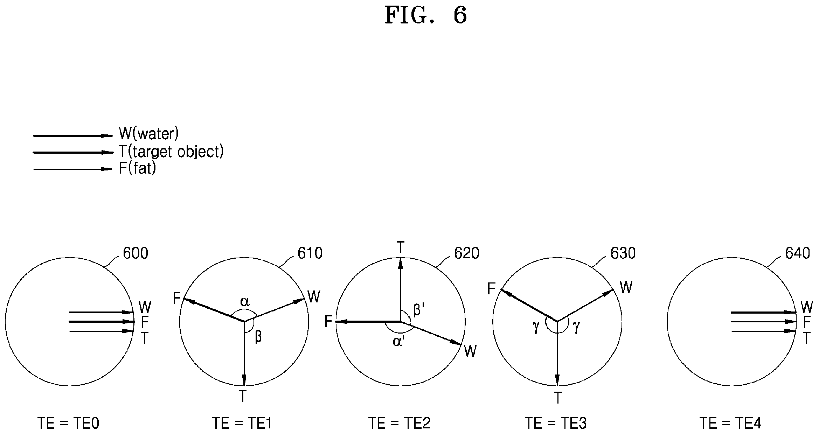

Referring to Equations (1) and (2) and Tables (1) and (2), the extent of a frequency shift $\Delta f$ in the part of interest into which the contrast agent is injected may vary depending on the type and concentration of contrast agent. Referring to the frequency spectrum 400 of FIG. 4, as the frequency shift $\Delta f$ in the part of interest 430 occurs to a greater extent, a frequency difference between the water 410 and the part of interest 430 becomes greater, and thus, a phase difference therebetween becomes greater. As the phase difference between the water 410 and the part of interest 430 becomes greater, it is easy to individually separate MR signals respectively originating from the water 410 and the part of interest 430 based on echo signals, as will be described in more detail below with reference to FIGS. 6 through 9.

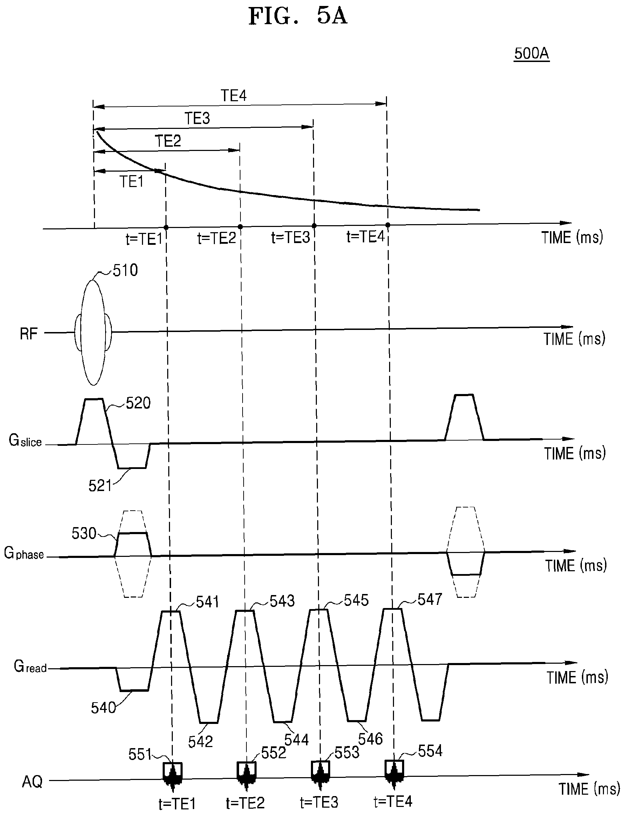

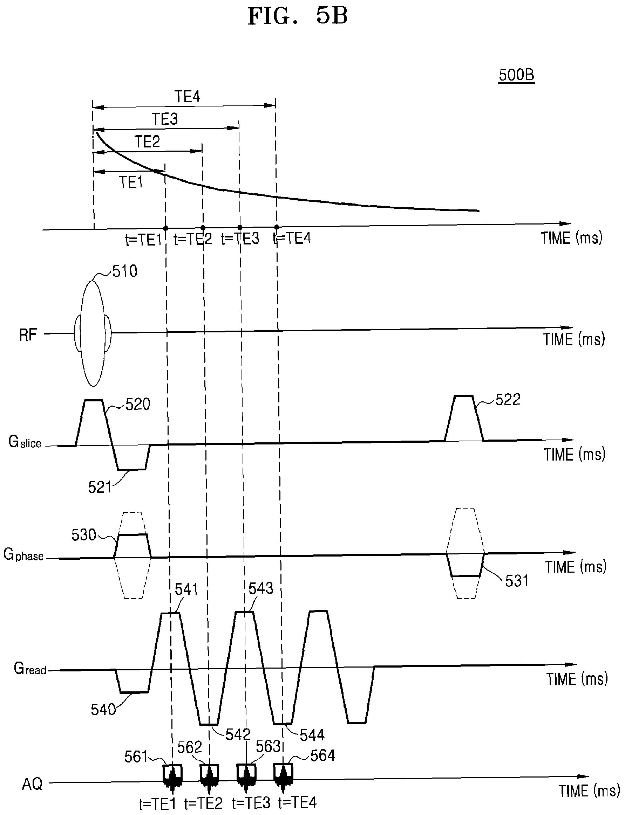

FIGS. 5A and 5B are timing diagrams for pulse sequence 500A and pulse sequence 500B performed by the MRI apparatus 200 to acquire echo signals, according to embodiments. The pulse sequences 500A and 500B shown in FIGS. 5A and 5B are merely examples, and pulse sequences generated by the MRI apparatus 200 are not limited to the pulse sequences 500A and 500B.

Referring to FIG. 5A, the pulse sequence 500A is a three-dimensional (3D) steady state free precession(SSFP) gradient echo sequence, and the MRI apparatus 200 may acquire a plurality of echo signals at a plurality of different TEs, for example TE 551, TE 552, TE 553, and TE 554 based on the pulse sequence 500A. The controller 220 of the MRI apparatus 200 may control the RF coil 214 to transmit an RF excitation pulse 510 to a part of interest into which a contrast agent is injected based on the pulse sequence 500A. Simultaneously with application of the RF excitation pulse 510 to the object, the gradient magnetic field generator 212 may apply a slice encoding gradient 520 to the part of interest of the object to excite a slice volume, and thereafter apply a slice encoding gradient 521 for encoding information about a position in a slice direction to the part of interest.

Furthermore, the gradient magnetic field generator 212 may apply a phase encoding gradient 530 for encoding position information to the part of interest, together with a frequency encoding gradient 540 that is in the opposite direction to the phase encoding gradient 530.

The controller 220 may calculate a TE at which a phase difference between each of water, fat, and the part of interest of an object reaches a same value as a preset phase angle and control the gradient magnetic field generator 212 to apply a frequency encoding gradient 541 in a positive direction to the part of interest in order to acquire a first echo signal at a first TE TE1.

Subsequently, the controller 220 may control the gradient magnetic field generator 212 to apply a frequency encoding gradient 542 having the same magnitude as but an opposite sign to the applied frequency encoding gradient 541 in order to remove dephasing of spins caused by the frequency encoding gradient 541.

The controller 220 may calculate again a second TE TE2 at which a phase difference between each of the water, the fat, and the part of interest is equal to a preset phase angle and control the gradient magnetic field generator 212 to apply a frequency encoding gradient 543 in the positive direction to the part of interest in order to acquire a second echo signal at the second TE TE2. The controller 220 may thereafter apply a frequency encoding gradient 544 that is in the opposite direction to the applied frequency encoding gradient 543 to the part of interest.

By repeating the above-described process, the controller 220 may control the gradient magnetic field generator 212 to apply frequency encoding gradient 541, frequency encoding gradient 543, frequency encoding gradient 545, and frequency encoding gradient 547 in the positive direction to the part of interest at the first through fourth TEs TE1 through TE4

The image processor 230 may respectively acquire first through fourth echo signals at first through fourth TEs TE1 through TE4. In an embodiment, the image processor 230 may acquire the first echo signal during a first echo signal acquisition time period 551. Similarly, the image processor 230 may respectively acquire the second through fourth echo signals during second through fourth echo signal acquisition time periods 552 through 554 at the second through fourth TEs TE2 through TE4.

The controller 220 may control the image processor 230 to acquire a plurality of echo signals by repeatedly performing application of the RF excitation pulse 510 and application of slice encoding gradient 520 and slice encoding gradient 521, phase encoding gradient 530, and frequency encoding gradients 540 through 547. The image processor 230 may acquire k-space data from the acquired plurality of echo signals and generate MR image data by performing an inverse Fourier transform on the k-space data.

Although the pulse sequence 500A is used to acquire a total of four echo signals across a total of four TEs (TE1, TE2, TE3, and TE4), this is merely an example. According to an embodiment, when one RF excitation pulse is applied to the part of interest, the MRI apparatus 200 may acquire at least three echo signals during at least three TEs.

Referring to FIG. 5B, the MRI apparatus 200 may acquire a plurality of echo signals during a plurality of different echo signal acquisition time periods 561 through 564 based on a pulse sequence 500B.

The pulse sequence 500B shown in FIG. 5B is the same as the pulse sequence 500A except that an echo signal can be acquired by applying even frequency encoding gradient 542 and even frequency encoding gradient 544 in a negative direction, opposite to the positive direction. Thus, descriptions that are already provided above with respect to FIG. 5A will not be repeated below.

Following application of a frequency encoding gradient 540 that is in the opposite direction to an applied phase encoding gradient 530 to a part of interest of an object, the controller 220 may control the gradient magnetic field generator 212 to apply a frequency encoding gradient 541 in the positive direction to the part of interest. The image processor 230 may acquire a first echo signal during a first echo signal acquisition time period 561 at a first TE TE1.

Thereafter, the controller 220 may calculate a second TE TE2 at which a phase difference between each of water, fat, and a part of interest is equal to a preset phase angle and control the gradient magnetic field generator 212 to apply a frequency encoding gradient 542 in a negative direction, opposite to the applied frequency encoding gradient 541, to the part of interest in order to acquire a second echo signal at the second TE TE2. The image processor 230 may acquire the second echo signal during a second echo signal acquisition time period 562 at the second TE TE2 when the frequency encoding gradient in the negative direction is applied.

Similarly, the controller 220 may calculate third and fourth TEs TE3 and TE4 and control the gradient magnetic field generator 212 to respectively apply frequency encoding gradients 543 and 544 in the positive and negative directions to the part of interest at the third and fourth TEs TE3 and TE4. The image processor 230 may respectively acquire third and fourth echo signals during a third echo signal acquisition time period 563 at the third TE TE3 and during a fourth echo signal acquisition time period 564 at the fourth TE TE4.

The pulse sequence 500B shown in FIG. 5B may shorten a time interval between the first and second TEs TE1 and TE2 compared to the pulse sequence 500A described with reference to FIG. 5A, thereby reducing the total scan time.

FIG. 6 is a schematic diagram for explaining phase differences among water, fat, and a target object T in pieces of image data reconstructed from echo signals respectively acquired by the MRI apparatus 200 at a plurality of TEs, according to an embodiment. In this case, the target object T means a part of interest that has undergone a resonance frequency shift due to injection of a contrast agent.

Referring to FIG. 6, first image data 610, second image data 620, third image data 630, and fourth image data 640 are complex image data in an image space obtained by digitizing echo signals respectively acquired at first through fourth TEs TE1 through TE4. Reference image data 600 is generated from an echo signal at TE=0 when a phase difference between each of water W, fat F, and the target object T is 0.

If there is no systematic phase error nor phase error due to inhomogeneity in a main magnetic field $B_0$, signal intensities $S_i$ for the first through fourth image data 610, 620, 630, and 640 may be determined based on Equation (4) below:

$$S_i = W + F * exp^{ia} + T * exp^{ib} \qquad \ldots (4)$$

where $S_i$ is a signal intensity for image data, W, F, and T are respectively signal intensities of water, fat, and a target object, and a and b are respectively phase differences, for example phase angles, between the water and the fat and between the water and the target object.

Since resonance frequency differences exist among the water W, the fat F, and the target object T, each of the water W, the fat F, and the target object T has a different phase difference therebetween at a plurality of TEs TE1 through TE4, respectively, as an RF excitation pulse is applied.

The first image data 610 is complex image data acquired at the first TE TE1 during which a phase difference between the water W and the fat F is equal to a first phase angle $\alpha$, and a phase difference between the water W and the target object T is equal to a second phase angle $\beta$. A signal intensity $S_1$ for the first image data 610 may be determined by using Equation (5) below:

$$S_1 = W + F * exp^{i\alpha} + T * exp^{i\beta} \qquad \dots (5)$$

In the same manner, the second image data 620 is complex image data acquired at the second TE TE2 during which a phase difference between the water W and the fat F is equal to a first phase angle $\alpha'$, and a phase difference between the water W and the target object T is equal to a second phase angle $\beta'$. A signal intensity $S_2$ for the second image data 620 may be determined by using Equation (6) below:

$$S_2 = W + F * exp^{i\alpha'} + T * exp^{i\beta'} \qquad \dots (6)$$

The third image data 630 is complex image data acquired at the third TE TE3 during which phase differences between the water W and the fat F and between the water W and the target object T are both equal to a third phase angle $\gamma$. A signal intensity $S_3$ for the third image data 630 may be determined by using Equation (7) below:

$$S_3 = W + F * exp^{i\gamma} + T * exp^{i\gamma} \qquad \dots (7)$$

The fourth image data 640 may be complex image data acquired at the fourth TE TE4 during which the water W, the fat F, and the target object T are all in phase. Since phase differences among the water W, the fat F, and the target object T are 0, a signal intensity $S_{\backslash 4}$ for the fourth image data 640 may be determined by using Equation (8) below:

$$S_4 = W + F + T \qquad \dots (8)$$

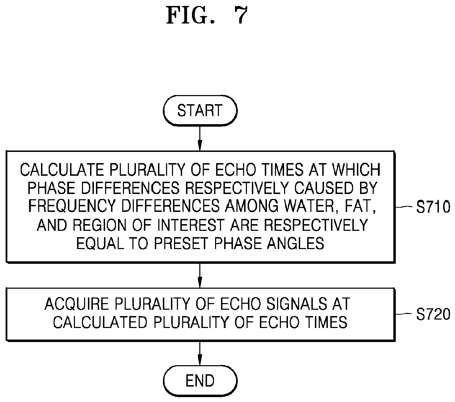

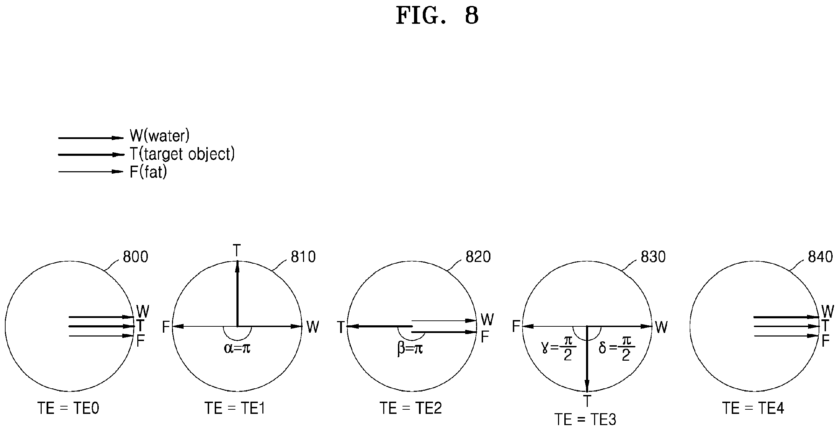

The MRI apparatus 200 may individually separate pieces of image data regarding the water W, the fat, and the target object T by performing a mathematical operation, e.g., by simultaneously solving complex equations shown in Equations (5) through (8) above. In an embodiment, the MRI apparatus 200 may calculate a plurality of TEs at which phase differences among the water W, the fat F, and the target object T are respectively equal to preset phase angles, eliminate image data regarding the water W and the fat from echo signals acquired at the plurality of TEs, and separate only image data regarding the target object T, as will be described in more detail with reference to FIGS. 7 and 8.

FIG. 7 is a flowchart of a method, performed by the MRI apparatus 200, of acquiring a plurality of echo signals according to an embodiment, and FIG. 8 is a schematic diagram for explaining phase differences among water W, fat F, and a target object T in pieces of image data including first image data 810, second image data 820, third image data 830, and fourth image data 840 respectively acquired by the MRI apparatus 200 at a plurality of TEs, according to an embodiment.

Referring to FIG. 7, the MRI apparatus 200 calculates a plurality of TEs at which phase differences respectively caused by frequency differences among water, fat, and a part of interest of an object are equal to their corresponding preset phase angles at operation S710. According to an embodiment, a resonance frequency shift may occur in the part of interest into which a contrast agent is injected and therefore induce a phase difference between the water and fat.

MRI apparatus 200 respectively acquires a plurality of echo signals at the calculated plurality of TEs at operation S720. According to an embodiment, the MRI apparatus 200 may acquire complex image data in an image space by digitizing the plurality of echo signals.

Referring to FIG. 8, reference image data 800 is image data of an echo signal acquired at TE=0 when a phase difference between each of the water W, the fat F, and the target object T is 0.

Referring to first image data 810, the MRI apparatus 200 may calculate a TE at which a phase difference $\alpha$ between the water W and the fat F is equal to a first phase angle $\pi$ and determine the calculated TE as a first TE TE1.

Similarly, referring to second image data 820, the MRI apparatus 200 may calculate a TE at which a phase difference $\beta$ between either of the water W and the fat F and the target object T is equal to a second phase angle $\pi$ and determine the calculated TE as a second TE TE2.

Referring to third image data 830, the MRI apparatus 200 may calculate a TE at which a phase difference $\gamma$ between the water W and the target object T is the same as a phase difference $\delta$ between the fat F and the target object T and the phase differences $\gamma$ and $\delta$ are all equal to $\pi/2$, and determine the calculated TE as a third TE TE3.

Referring to fourth image data 840, the MRI apparatus 200 may calculate a TE at which the water W, the fat F, and the target object T are all in phase and determine the calculated TE as a fourth TE TE4.

Signal intensities $S_{TE1}$ through $S_{TE4}$ for the first through fourth image data 810, 820, 830, and 840 may be determined by using Equation (9) below:

$$S_{TE1} = W - F + i * T$$

$$S_{TE2} = W + F - T$$

$$S_{TE3} = W - F - i * T$$

$$S_{TE4} = W + F + T \qquad \ldots (9)$$

In an embodiment, the MRI apparatus 200 may separate each of pieces of image data regarding the water W, the fat F, and the target object T by performing a mathematical operation of solving four complex simultaneous equations included in Equation (9) above. For example, the MRI apparatus 200 may individually separate pieces of image data regarding the water W, the fat F, and the target object T by using Equation (10) below:

$$S_5 = \frac{S_{TE1} + S_{TE3}}{2} = W - F$$

$$S_6 = \frac{S_{TE2} + S_{TE4}}{2} = W + F$$

$$\frac{S_5 + S_6}{2} = W$$

$$\frac{S_6 - S_5}{2} = F$$

$$\frac{S_{TE4} - S_{TE2}}{2} = T \qquad \ldots (10)$$

Referring to Equation (10) above, when two complex equations that respectively define the signal intensities $S_{TE1}$ and $S_{TE3}$ for the first and third image data 810 and 830 at the first and third TEs TE1 and TE3 are added and the sum is divided by 2, then fifth image data $S_5$ (W-F) is obtained by eliminating the fat F from the water W. In the same manner, when two complex equations that respectively define the signal intensities $S_{TE2}$ and $S_{TE4}$ for the first and third image data 820 and 840 at the second and fourth TEs TE2 and TE4 are added and the sum is divided by 2, then sixth image data S6 (W+F) is obtained by adding together the water W and the fat F.

By performing an arithmetic operation of adding the fifth image data S5 to the sixth image data S6, it is possible to separate only image data of the water W. Similarly, by performing an arithmetic operation of subtracting the fifth image data S5 from the sixth image data S6, it is possible to separate only image data of the fat F.

Furthermore, by subtracting the signal intensity $S_{TE2}$ for the second image data 820 at the second TE TE2 from the signal intensity $S_{TE4}$ for the fourth image data 840 at the fourth TE TE4, it is possible to separate only image data of the target object T.

The embodiment shown in FIG. 8 and the simultaneous signal intensity equations for the first through fourth image data 810, 820, 830, and 840 described with reference to Equations (9) and (10) above are merely examples for explaining a method of individually separating pieces of image data regarding the water W, the fat F, and the target object T. Thus, equations used by the MRI apparatus 200 to separate each pieces of image data regarding the water W, the fat F, and the target object T are not limited to Equations (9) and (10).

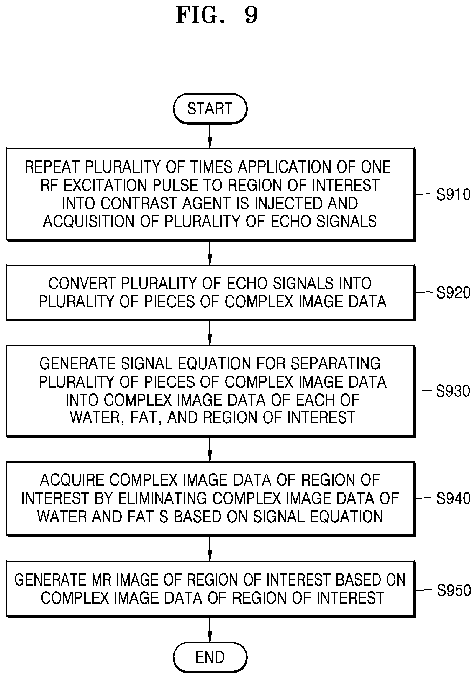

FIG. 9 is a flowchart of a method, performed by the MRI apparatus 200, of generating an MR Image of a part of interest according to an embodiment.

The MRI apparatus 200 repeats a plurality of times an operation of applying one RF excitation pulse to a part of interest of an object into which a contrast agent is injected and acquiring a plurality of echo signals at operation S910. Operation S910 is performed by repeating operations S310 and S320 described with reference to FIG. 3 a plurality of times. In an embodiment, the number of times that one RF excitation pulse is applied to a part of interest and a plurality of echo signals are acquired may mean the number of times that the echo signals can completely fill ky lines in a k-space.

The MRI apparatus 200 converts the plurality of echo signals into a plurality of pieces of complex image data at operation S920. In an embodiment, the MRI apparatus 200 may digitize the acquired plurality of echo signals and generate a plurality of pieces of complex image data by performing an inverse Fourier transform. etc.

The MRI apparatus 200 generates a signal equation for separating the plurality of pieces of complex image data into complex image data of water, complex image data of fat, and complex image data of the part of interest at operation S930. According to an embodiment, the MRI apparatus 200 may generate a signal equation for simultaneously solving complex equations that respectively define signal intensities for complex image data acquired at a plurality of TEs and for performing mathematical operations such as addition, subtraction, etc. In an embodiment, the image processor 230 of the MRI apparatus 200 may be implemented as a CPU, a microprocessor, or an ASIC and generate at high speed a signal equation to find unknown numbers, i.e., image data regarding the water, the fat, and the part of interest as solutions to a plurality of complex equations.

The MRI apparatus 200 acquires the complex image data of the part of interest by eliminating the complex image data of the water and fat based on the signal equation at operation S940. According to an embodiment, the image processor 230 may eliminate image data of the water and fat from a plurality of pieces of image data by performing a mathematical operation such as solving simultaneous equations based on the generated signal equation.

The MRI apparatus 200 generates an MR image of the part of interest based on the complex image data of the part of interest at operation S950. In an embodiment, the image processor 230 may acquire k-space data from the complex image data of the part of interest and generate an MR image of the part of interest by performing a reconstruction process such as an inverse Fourier transform.

Embodiments may be implemented through non-transitory computer-readable recording media having recorded thereon computer-executable instructions and data. The instructions may be stored in the form of program codes, and when executed by a processor, generate a predetermined program module to perform a specific operation. Furthermore, when being executed by the processor, the instructions may perform specific operations according to the embodiments.

While one or more embodiments have been described with reference to the figures, it will be understood by those of ordinary skill in the art that various changes in form and details may be made therein without departing from the spirit and scope of the present disclosure as defined by the following claims. Accordingly, the above embodiments and all aspects thereof are examples only and are not limiting.

**Claims**

1. A method of acquiring a magnetic resonance (MR) image by injecting a contrast agent into a region of interest of an object, the method comprising:

   applying one radio frequency (RF) excitation pulse to the region of interest after the region of interest has undergone a frequency shift caused by the injected contrast agent;
   acquiring a plurality of echo signals at a plurality of echo times, the plurality of echo times being determined based on phase differences among water, fat, and the region of interest; and
   separating, based on the acquired plurality of echo signals, MR signals originating respectively from the water, the fat, and the region of interest.

2. The method of claim 1, wherein the acquiring of the plurality of echo signals comprises acquiring at least three echo signals across at least three echo times.

3. The method of claim 1, wherein the acquiring of the plurality of echo signals comprises:

   determining the plurality of echo times at which phase differences caused by frequency differences among the water, the fat, and the region of interest reach predetermined phase difference values; and
   acquiring the plurality of echo signals at the calculated plurality of echo times.

4. The method of claim 1, further comprising repeating the applying of the one RF excitation pulse and the acquiring of the plurality of echo signals a plurality of times.

5. The method of claim 1, wherein the separating of the MR signals comprises:

   generating complex image data of the water, complex image data of the fat, and complex image data of the region of interest by reconstructing the acquired plurality of echo signals;
generating a signal equation for individually separating the complex image data of the water, the complex image data of the fat, and the complex image data of the region of interest; and
eliminating the complex image data of the water and the complex image data of the fat based on the signal equation; and
acquiring the complex image data of the region of interest.

6. An apparatus for acquiring a magnetic resonance (MR) image by injecting a contrast agent into a region of interest of an object, the apparatus comprising:

a radio frequency (RF) coil configured to apply one RF excitation pulse to the region of interest after the region of interest has undergone a frequency shift caused by the injected contrast agent;
a controller configured to determine a plurality of echo times based on phase differences among water, fat, and the region of interest; and
an image processor configured to acquire a plurality of echo signals at the plurality of echo times and separate, based on the acquired plurality of echo signals, MR signals originating respectively from the water, the fat, and the region of interest.

7. The apparatus of claim 6, wherein the image processor is further configured to acquire at least three echo signals across at least three echo times.

8. The apparatus of claim 6, wherein the controller is further configured to calculate the plurality of echo times at which phase differences caused by frequency differences among the water, the fat, and the region of interest reach predetermined phase difference values, and
wherein the image processor is further configured to acquire the plurality of echo signals at the calculated plurality of echo times.

9. The apparatus of claim 8, wherein the controller is further configured to:

determine a first echo time at which a phase difference between the water and the fat is equal to a first phase angle;
determine a second echo time at which a phase difference between the water and the region of interest is equal to a second phase angle;
determine a third echo time at which the phase difference between the water and the region of interest is equal to a phase difference between the fat and the region of interest; and
determine a fourth echo time at which the water, the fat, and the region of interest are all in phase.

10. The apparatus of claim 9, wherein the controller is further configured to determine the first echo time as an echo time at which the first phase angle is $\pi$.

11. The apparatus of claim 9, wherein the controller is further configured to determine the second echo time as an echo time at which the second phase angle is $\pi$.

12. The apparatus of claim 9, wherein the controller is further configured to determine the third echo time as an echo time at which the phase difference between the water and the region of interest is equal to $\pi/2$ and the phase difference between the fat and the region of interest is equal to $\pi/2$.

13. The apparatus of claim 6, wherein the RF coil is further configured to repeatedly apply the one RF excitation pulse a plurality of times and the controller is further configured to repeatedly acquire the plurality of echo signals a plurality of times.

14. The apparatus of claim 6, wherein the image processor is further configured to:

generate complex image data of the water, complex image data of the fat, and complex image data of the region of interest by reconstructing the acquired plurality of echo signals;
generate a signal equation for individually separating the complex image data of the water, the complex image data of the fat, and the complex image data of the region of interest;
acquire the complex image data of the region of interest by eliminating the complex image data of the water and the complex image data of the fat based on the signal equation; and

reconstruct an MR image of the region of interest from the complex image data of the region of interest.

15. A non-transitory computer-readable recording medium having recorded thereon at least one program which, when executed by a processor, causes the processor to perform the method of claim 1.

FIG. 1

EP 3 358 362 A1

# FIG. 2

# FIG. 3

```
( START )
    │
    ▼
┌─────────────────────────────────────────┐
│  APPLY ONE RF EXCITATION PUSLE TO OBJECT │ ── S310
│   INTO WHICH CONTRAST AGENT IS INJECTED  │
└─────────────────────────────────────────┘
    │
    ▼
┌─────────────────────────────────────────┐
│  ACQUIRE PLURALITY OF ECHO SIGNALS AT    │
│  PLURLAITY OF DIFFERENT TES BASED ON     │ ── S320
│  PHASE DIFFERENCES AMONG WATER, FAT,     │
│  AND REGION OF INTEREST                  │
└─────────────────────────────────────────┘
    │
    ▼
┌─────────────────────────────────────────┐
│  SEPARATE MR SIGNALS RESPECTIVELY        │
│  ORIGINATING FROM WATER, FAT, AND        │ ── S330
│  REGION OF INTEREST BASED ON ACQUIRED    │
│  PLURALITY OF ECHO SIGNALS               │
└─────────────────────────────────────────┘
    │
    ▼
 ( END )
```

# FIG. 4

# FIG. 5A

500A

# FIG. 5B

# FIG. 6

EP 3 358 362 A1

W(water) →
T(target object) →
F(fat) →

600
W
F
T
TE = TE0

610
F α W
β
T
TE = TE1

620
T
F β'
α'
W
TE = TE2

630
F γ γ W
T
TE = TE3

640
W
F
T
TE = TE4

# FIG. 7

```
          ┌─────────┐
          │  START  │
          └─────────┘
               │
               ▼
┌──────────────────────────────────────────┐
│  CALCULATE PLURALITY OF ECHO TIMES AT WHICH │
│  PHASE DIFFERENCES RESPECTIVELY CAUSED BY   │ ─── S710
│  FREQUENCY DIFFERENCES AMONG WATER, FAT,    │
│  AND REGION OF INTEREST ARE RESPECTIVELY    │
│  EQUAL TO PRESET PHASE ANGLES               │
└──────────────────────────────────────────┘
               │
               ▼
┌──────────────────────────────────────────┐
│     ACQUIRE PLURALITY OF ECHO SIGNALS AT    │ ─── S720
│     CALCULATED PLURALITY OF ECHO TIMES      │
└──────────────────────────────────────────┘
               │
               ▼
          ┌─────────┐
          │   END   │
          └─────────┘
```

# FIG. 8

W(water)
T(target object)
F(fat)

TE = TE0

800

TE = TE1

810

$\alpha = \pi$

T

W

F

TE = TE2

820

$\beta = \pi$

T

W

F

TE = TE3

830

$\gamma = \dfrac{\pi}{2}$

$\delta = \dfrac{\pi}{2}$

W

T

F

TE = TE4

840

W
T
F

# FIG. 9

START

REPEAT PLURALITY OF TIMES APPLICATION OF ONE RF EXCITATION PULSE TO REGION OF INTEREST INTO CONTRAST AGENT IS INJECTED AND ACQUISITION OF PLURALITY OF ECHO SIGNALS — S910

CONVERT PLURALITY OF ECHO SIGNALS INTO PLURALITY OF PIECES OF COMPLEX IMAGE DATA — S920

GENERATE SIGNAL EQUATION FOR SEPARATING PLURALITY OF PIECES OF COMPLEX IMAGE DATA INTO COMPLEX IMAGE DATA OF EACH OF WATER, FAT, AND REGION OF INTEREST — S930

ACQUIRE COMPLEX IMAGE DATA OF REGION OF INTEREST BY ELIMINATING COMPLEX IMAGE DATA OF WATER AND FAT S BASED ON SIGNAL EQUATION — S940

GENERATE MR IMAGE OF REGION OF INTEREST BASED ON COMPLEX IMAGE DATA OF REGION OF INTEREST — S950

END

Europäisches
Patentamt

European
Patent Office

Office européen
des brevets

## EUROPEAN SEARCH REPORT

Application Number

EP 18 15 4464

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2013/089271 A1 (BOERNERT PETER [DE] ET AL) 11 April 2013 (2013-04-11)<br>* paragraphs [0006] - [0029], [0039], [0045], [0047], [0051], [0052], [0058] - [0067]; claim 1 * | 1-4,6-8,<br>13,15<br>5,9-12,<br>14 | INV.<br>G01R33/48 |
| A | US 6 147 492 A (ZHANG WEIGUO [US] ET AL) 14 November 2000 (2000-11-14)<br>* column 9, line 5 - column 10, line 30 * | 1,6,8,9 | |
| A | WO 2015/159172 A1 (KONINKL PHILIPS NV [NL]; UNIV TEXAS SOUTHWESTERN MED CT [US]) 22 October 2015 (2015-10-22)<br>* claim 1 * | 1,6 | |

TECHNICAL FIELDS
SEARCHED        (IPC)

G01R

The present search report has been drawn up for all claims

| Place of search | Date of completion of the search | Examiner |
|---|---|---|
| Munich | 11 June 2018 | Skalla, Jörg |

## ANNEX TO THE EUROPEAN SEARCH REPORT
## ON EUROPEAN PATENT APPLICATION NO.

EP 18 15 4464

This annex lists the patent family members relating to the patent documents cited in  the above-mentioned European search report.
The members are as contained in the European Patent Office EDP file on
The European Patent Office is in no way liable for these particulars which are merely given for the purpose of information.

11-06-2018

| Patent document cited in search report | | Publication date | Patent family member(s) | | Publication date |
|---|---|---|---|---|---|
| US 2013089271 | A1 | 11-04-2013 | CN | 102959388 A | 06-03-2013 |
| | | | GB | 2495447 A | 10-04-2013 |
| | | | RU | 2013103063 A | 27-07-2014 |
| | | | US | 2013089271 A1 | 11-04-2013 |
| | | | WO | 2011161566 A1 | 29-12-2011 |
| US 6147492 | A | 14-11-2000 | JP | 4444413 B2 | 31-03-2010 |
| | | | JP | 2000135206 A | 16-05-2000 |
| | | | US | 6147492 A | 14-11-2000 |
| WO 2015159172 | A1 | 22-10-2015 | CN | 106456046 A | 22-02-2017 |
| | | | EP | 3131458 A1 | 22-02-2017 |
| | | | JP | 2017511227 A | 20-04-2017 |
| | | | US | 2017027472 A1 | 02-02-2017 |
| | | | WO | 2015159172 A1 | 22-10-2015 |

EPO FORM P0459

For more details about this annex : see Official Journal of the European Patent Office, No. 12/82

**EP 3 358 362 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020170014384